(19) **Europäisches Patentamt / European Patent Office / Office européen des brevets**

(11) **EP 2 120 881 B1**

(12) **EUROPEAN PATENT SPECIFICATION**

(45) Date of publication and mention of the grant of the patent:
**07.08.2019 Bulletin 2019/32**

(21) Application number: **08725289.6**

(22) Date of filing: **06.02.2008**

(51) Int Cl.:
*A61K 9/16* (2006.01)     *A61K 9/50* (2006.01)
*A61K 31/138* (2006.01)

(86) International application number:
**PCT/US2008/001639**

(87) International publication number:
**WO 2008/103245 (28.08.2008 Gazette 2008/35)**

(54) **METHOD OF DETERMINING THE WEIGHT OF THE COATING TO BE APPLIED TO FORM A CONTROLLED RELEASE DOSAGE FORM**

VERFAHREN ZUR BESTIMMUNG DES GEWICHTS EINER ZUR FORMUNG EINER DOSIERUNGSFORM MIT GESTEUERTER FREISETZUNG ANGEBRACHTEN BESCHICHTUNG

PROCÉDÉ DE DÉTERMINATION DU POIDS DE REVÊTEMENT À APPLIQUER POUR FORMER UNE FORME POSOLOGIQUE À LIBÉRATION CONTRÔLÉE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **23.02.2007 US 903224 P**

(43) Date of publication of application:
**25.11.2009 Bulletin 2009/48**

(73) Proprietor: **GLATT AIR TECHNIQUES, INC.**
**Ramsey, NJ 07446-1288 (US)**

(72) Inventors:
- **RUBINO, Orpapin, P.**
  **Towaco, NJ 07082 (US)**
- **JONES, David**
  **Ramsey, NJ 07446 (US)**
- **FEMIA, Robert, A.**
  **Kinnelon, NJ 07405 (US)**
- **MUELLER, Oliver, W.**
  **Kinnelon, NJ 07405 (US)**
- **RANGUNATHAN, Narayan**
  **West Nyack, NY 10994 (US)**

- **POLLINGER, Norbert**
  **79379 Mullheim (DE)**
- **PRASCH, Armin**
  **79249 Merzhausen (DE)**
- **ETTNER, Anne**
  **79400 Wollbach-kandern (DE)**

(74) Representative: **Henkel, Breuer & Partner**
**Patentanwälte**
**Brienner Strasse 1**
**80333 München (DE)**

(56) References cited:
**WO-A1-02/072074     WO-A1-2005/115340**
**CA-C- 1 293 449     US-A- 5 527 545**
**US-A1- 2006 099 259**

- **OZTURK A G ET AL: "Mechanism of release from pellets coated with an ethylcellulose-based film", JOURNAL OF CONTROLLED RELEASE, ELSEVIER, AMSTERDAM, NL, vol. 14, no. 3, 1 December 1990 (1990-12-01), pages 203-213, XP025551792, ISSN: 0168-3659, DOI: 10.1016/0168-3659(90)90160-U [retrieved on 1990-12-01]**

**Description**

BACKGROUND OF THE INVENTION

The formulation of controlled release membranes by the use of liquid coating systems is well known. In the prior art it is known that it is difficult to consistently manufacture batches of membrane coated products that have the same release profiles both in vitro and in vivo. This is a particular problem with drugs such as beta blockers that have a narrow therapeutic index which makes it essential that the vivo and in vitro release profiles are consistent from batch to batch. For this reason, the art has developed sophisticated apparatus and process controls for the rate, temperature, humidity and times that are employed for forming semi-permeable membranes on solid particles that contain active chemicals such as pharmaceuticals, seeds, fertilizers and the like. Even with careful process controls, it is sometimes necessary to reprocess or discard multiparticulate controlled release products due to a lack of conformance with established release rate profiles.

The amount of a coating agent to provide a desired therapeutic effect may be determined using conventional experimental techniques that measure the release rate of a pharmaceutical from a particular membrane system by trial and error using in vitro and in vivo procedures. These methods are based on the use of preset process controls that do not take into account any variations in the size or size distribution curve for the multiparticulate particles that are to be coated.

In Aqueous Polymeric Coatings for Pharmaceutical Dosage Forms, J.W. McGinity Ed., Marcel Dekker. Inc., NY (1989), pp. 350-355, it was disclosed that surface rugosity might have a possible effect on the release rate of a drug contained in membrane coated particles. The concept that was disclosed was that particles of equivalent sphere diameters would have different surface areas depending on the smoothness of the surface of the spheres. No information was given as to how to reproducibly prepare batches of coated spherical particles that will release active ingredients at reproducible rates. In Pharmaceutical Pelletization Technology, Isaac Ghebre-Sellassie Ed., Marcel Dekker, Inc., NY (1989), pp. 246-252, it was disclosed that rough pellets have a greater surface area than smooth pellets and when the same weight of a coating is applied to separate batch of smooth pellets and a separate batch of rough pellets, the smooth pellets will exhibit a faster rate of release than the rough pellets.

U.S. 5,527,545 describes a liquid suspension of enteric coated pellets of naproxen, which is a drug having a low solubility in water. In the course of making the multiple coated naproxen pellets, the surface area of a batch of pellets is used as a basis apply a constant amount of coating so that the coating is directly proportional to the surface area. No drug other than naproxen is mentioned by U.S. 5,527,545 and the naproxen pellets range in size from 50 to 500$\mu$m and each pellet is provided with four separate coatings at least one of which is an enteric coating. U.S. 4,138,475 discloses a specific propranolol controlled release formulation.

The present invention provides a reproducible method for making controlled release pellets containing a beta-blocker compound where a single membrane is applied to a pelleted formulation, where the weight of the coating is based on the surface area of the pellet and the weight of the coating is determined by selecting a coating weight that is proportional to the surface area of the beta-blocker pellets, based on a weight per surface area of the beta-blocker pellets, previously determined *in vivo* or *in vitro* release profile. The present invention is particularly applicable to beta-blockers such as propranolol which have a high solubility in water.

SUMMARY OF THE INVENTION

The invention provides a method of coating substantially spherical particles comprising a beta-blocker compound where the pellets are substantially free of surface defects, with a membrane forming coating to provide a membrane on said substantially spherical particles that controls the release of an active compound from said substantially spherical particle at a predetermined level. The method comprises the following steps:

(a) determining the surface area of a weighed sample of said substantially spherical particles in a first batch of substantially spherical particles based on the particle size distribution and density;

(b) determining the weight of coating material per surface area unit of said substantially spherical particles that will provide a coating level of a composition for said substantially spherical particles of step (a) that will provide a desired release profile in vivo;

(c) determining the surface area of a new batch of substantially spherical particles containing the same active compound that was contained in the substantially spherical particles that were coated in step(b);

(d) coating said new batch of substantially spherical particles with the coating applied in step (b) to provide a coating on said new batch of substantially spherical particles, wherein said coating on said new batch of spherical particles has the substantially the same weight of coating material per surface area unit of substantially spherical particles as determined in step (b).

Accordingly, it is a primary object of the invention to provide a method of producing controlled release substantially spherical particles of a beta-blocker which have a consistent in vitro and in vivo release profiles.

It is also an object of the invention to provide a method manufacturing controlled release multiparticulate particles of a beta-blocker which avoids the necessity of reworking or discarding the coated particles because they fail to meet established release rate profiles.

These and other objects of the invention will become apparent from ther appended specification.

The term beta-blocker is used to describe those pharmaceutical compounds that are classified as beta-adrenergic receptor blocking agents such as propranolol, metoprolol, +timolol, oxpremolol, alpremolol, pindolol, sotalol, alebutolol, atenolol, and the like. The release rates are expressed in terms of weight percent of the total active compound in the dosage form which is released after a measured lapse of time in accordance with standard procedures that are well know in the art.

DETAILED DESCRIPTION OF THE INVENTION

In the practice of the present invention, it is contemplated that a multiparticulate controlled release formulation of a beta-blocker will have been made and tested for either a desired pharmacological effect or for the purpose of providing a formulation of the same beta-blocker that is bioequivalent to a previously approved controlled release formulation. Once a particular beta-blocker has been approved for full scale manufacturing, the present invention may be utilized to provide a coating parameter that will result in a method of manufacturing a multiparticulate formulation of the beta-blocker that has a consistent release profile.

The ratio of the weight of coating per unit surface area for a particular beta-blocker and a particular membrane based release controlling system will be unique for that product with regard to the particular release rate profile for that product whether it is in vivo or in vitro. The invention is particularly useful with thin coatings of controlled release membranes.

Once an operable system for making a multiparticulate controlled release formulation has been identified, the present invention may be utilized to provide a manufacturing method that will provide sufficient batch to batch uniformity that it will avoid the need to destroy particular batches which are out of specification due to failed release rate tests.

Thus, when a release controlling system is chosen for a particular beta-blocker, it mayl be experimentally determined which type of a membrane forming system and adjuvants will provide the desired controlled release system for the particular beta-blocker. The coating ratio that is determined for that particular system will be unique for that system and it will be necessary to determine a coating ratio for each beta-blocker release system. The invention is particularly useful when substantially spherical particles are made that are substantially free of surface defects. These spherical particles may be made using techniques such as seed coating using sugar seeds or other solid bead forming materials,(i.e microcrystalline cellulose pellets such as Cellets) spheronization, microtabletting techniques, or by the use of the CPS techniques disclosed in U.S.6,449,869; U.S.6,354,728; and U.S. 2004/0185111. The term "substantially spherical" is used to describe spherical particles useful in the present invention. These particles will have an aspect ratio, which is the ratio of the shortest axis of the particle to the longest axis of the particle, of from 0.2-1. It is to be understood that a perfect sphere will have an aspect ratio of 1.

After an operable formulation has been made using conventional coating techniques based on the use of membrane forming polymers that are applied to the spherical particles using conventional layering techniques and it has been decided to produce large scale quantities of such a formulation, the first step is to determine the surface area of the substantially spherical particles which comprise the beta-blocker, after they have been coated or layered with the beta-blocker. The specific surface area may be determined by methods such as measurement of the pellet coupled with a determination of the average particle size distribution, or by gas adsorption or air permeability. Pharmaceutical Pelletization Technology, Isaac Ghebre-Sellassie Ed., Marcel Dekker, Inc., NY (1989), pp. 246-252, discloses various techniques for determining the surface area of pellets.

The surface area is measured to provide a baseline of total surface area of the uncoated substantially spherical microparticles containing a beta-blocker, which are to be coated to give a particular release profile, when the multiparticulate composition is subsequently coated and tested to establish a release profile. The next step comprises determining the quantity of coating material, that is necessary to provide a controlled release multiparticulate beta-blocker formulation having the desired release profile, and then using the surface area and total weight (solids) that will provide the desired release profile to calculate the ratio of surface area to the weight (solids) of controlled release forming composition. The weight of the controlled release coating per unit of surface area is used to determine how much controlled release formulation is to be applied to the multiparticulate formulation of the beta-blocker.

A formula that may be used to calculate the specific surface area is

$$\text{Specific Surface area} \quad = \quad \frac{6}{\text{true density x mean volume surface diameter of the spheres.}}$$

The preferred coating weight is between about 0.05 to 3 mg/cm$^2$ and the especially preferred coating weight is between 0.1 to 1mg/cm$^2$ of surface area of multiparticulate particles. The coating conditions will vary depending on the materials that are employed and these conditions may be determined using conventional techniques.

The total applied coating weight is preferably from 1 to 30g/100g of starting material. and especially preferably from 1 to 10g/100g of starting material.

The particle sizes should be between 100 and 1400 microns in diameter, preferably from 600 to 1200 microns in diameter; and especially preferably from 700 to 1100 microns in diameter.

The particular controlled release coatings may be selected from those which are commerically available as FDA approved materials for use in pharmaceuticals when formulating a pharmaceutical or other compositions. Examples of useful materials are described in Aqueous Polymeric Coatings for Pharmaceutical Dosage Forms, J.W. McGinity Ed., Marcel Dekker. Inc. ,NY (1989), ppl-153. Generally, in the process of the invention, it has been found that unplasticized coatings that are not enteric coatings are preferred. Ethyl cellulose is a preferred coating material for use in the invention

Example I

A 70kg. batch of propranolol HCl core pellets (60% propranolol - 40% microcrystalline cellulose) were produced using a direct pelletization process (U.S.6,449,869; U.S.6,354,728; and U.S. 2004/0185111). The propranolol and the microcrystalline cellulose were prewetted and loaded into an apparatus as described in U.S. 6,449869 and U.S. 6,354,728. Water is applied until pellets of the desired size of approximately 600 to 1000 micron were formed. The pellets were dried in a fluid bed drier at a inlet air temperature of 60 °C. The pellets were coated in the GPCG-30 (Glatt Fluid Bed Processor, equipped with a 18" Wurster HS SRS (bottom spray) insert. The target coating level for propranolol core pellets was determined to be 0.41 mg/cm$^2$ to obtain desirable release profile. The coating polymers were ethyl cellulose and hypromellose in organic solvents.

Data from coating of five different Propranolol HCl core pellet batches were summarized in Table 1. Using specific surface area coating concept to adjust quantity of coating material for each batch of core pellets, results in reproducible dissolution profiles of the coated pellets. In all five coating batches, yield was greater than 97%.

Table 1:

| 1. Starting Materials | | | | | |
|---|---|---|---|---|---|
| 1.1 Propranolol HCl Core Pellets | | | | | |
| Batch # Propranolol Core Pellets GAT | A | B | C | D | E |
| Real density [g/cm3] | 1.322 | 1.3362 | 1.3379 | 1.3359 | 1.3348 |
| Sauter Diameter | 765.53 | 778.79 | 771.29 | 747.39 | 782.08 |
| Specific Surface Area [cm$^2$/g] | 59.3 | 57.7 | 58.1 | 58 | 57.5 |
| Theoretical Coating per kg Core pellets [g/kg] | 486.26 | 473.14 | 476.42 | 475.6 | 471.5 |
| Amount of liquid to be processed [kg] | 33.72 | 33.14 | 33.4 | 33.32 | 33.03 |
| 2. Process parameters | | | | | |
| Spray Time | n.a. | 157 | 160 | 156 | 167 |
| Drying Time | 5 + 5 | 10 | 10 | 10 | 10 |
| Spray pressure | 2.5 | 2.5 | 2.5 | 2.5 | 2.5 |
| Air volume [cm3/h] | 736-767 | 750 | 746-767 | 744-780 | 745-775 |
| Product Temperature during spraying(Min / Max) | 37.6-39.2 | 38.5-39.2 | 38.3-39.7 | 38.6-40.0 | 38.2-39.4 |
| 3. Coated Propranolol HCl Pellets Yield [%] | 99.17 | 97.26 | 97.55 | 97.43 | 97.5 |
| Coated Batch Numbers Dissolution Results | AC | BC | CC | DC | EC |
| Dissolution Propranolol HCl after 90 min [%] | 23.6 | 23.6 | 24.9 | 26.2 | 28.2 |
| Dissolution Propranolol HCL after 240 min [%] | 53.1 | 51.7 | 55.7 | 56.8 | 58.8 |
| Dissolution Propranolol HCL after 480 min [%] | 73.8 | 72.5 | 77.8 | 78.3 | 79.7 |

The Sauter diameter is determined by a laser light scattering method using a Malvern Particle Analyzer.

A preferred propranolol hydrochloride formulation will have the following release profile when tested in Apparatus 1, according to U.S.P.30, Method B for a delayed release dosage form using 900ml of pH 1.2 buffer solution for 1.5 hours during the acid stage using acceptance criteria given under Acceptance Table 3. For the buffer stage, 900ml of pH6.8 buffer solution is used for the remainder of the test. Samples are analyzed using UV absorption at 320nm with respect to a baseline drawn from 355nm through 340nm with a propranolol reference standard.

| Hours | Amount dissolved |
|-------|------------------|
| 1.5   | 15-30%           |
| 4     | 40-60%           |
| 8     | 65-80%           |

## Claims

1. A method of coating substantially spherical particles comprising a beta-blocker compound where the pellets are substantially free of surface defects, with a membrane forming coating to provide a membrane on said substantially spherical particles that controls the release of the active compound from said substantially spherical particle, said method comprising the following steps:

   (a) determining the surface area of a weighed sample of said substantially spherical particles comprising a beta-blocker compound in a first batch of substantially spherical particles based on the particle size distribution and density;
   (b) determining the weight of coating material per surface area unit of said substantially spherical particles that will provide a coating level of a composition for said substantially spherical particles of step (a) that will provide a desired release profile in vivo;
   (c) determining the surface area of a new batch of substantially spherical particles containing the same active compound that was contained in the substantially spherical particles that were coated in step (b);
   (d) coating said new batch of substantially spherical particles with the coating applied in step (b) to provide a coating on said new batch of substantially spherical particles, wherein said coating on said new batch of spherical particles has the substantially the same weight of coating material per surface area unit of substantially spherical particles as determined in step (b).

2. The method as defined in claim 1, wherein the applied total coating weight is between 0.05 to 3 mg/cm$^2$.

3. The method as defined in claim 2, wherein the applied total coating weight is between 0.1 to 1 mg/cm$^2$.

4. The method according to any preceding claim, wherein the particle sizes are between 100 and 1400 microns in diameter.

5. The method as defined in claim 4, wherein the particle sizes are between 600 and 1200 microns in diameter.

6. The method as defined in claim 5, wherein the particle sizes are between 700 and 1100 microns in diameter.

7. The method according to any preceding claim, wherein the beta blocker is selected from the group consisting of propranolol, metoprolol, timolol, oxpremolol, alpremolol, pindolol, sotalol, alebutolol and atenolol.

8. The method as defined in claim 7, wherein the beta-blocker is propranolol hydrochoride.

## Patentansprüche

1. Verfahren zum Beschichten von im Wesentlichen kugelförmigen Teilchen enthaltend eine Betablockerverbindung, wobei die Pellets im Wesentlichen frei von Oberflächendefekten sind, mit einer membranbildenden Beschichtung, um eine Membran auf den im Wesentlichen kugelförmigen Teilchen bereitzustellen, die die Freisetzung des Wirkstoffs aus den im Wesentlichen kugelförmigen Teilchen steuert, wobei das Verfahren die folgenden Schritte umfasst:

   (a) Bestimmen der Oberfläche einer abgewogenen Probe der im Wesentlichen kugelförmigen Teilchen enthaltend eine Beta-Blocker-Verbindung in einer ersten Charge von im Wesentlichen kugelförmigen Teilchen basierend auf der Teilchengrößenverteilung und - dichte;

(b) Bestimmen des Gewichts an Beschichtungsmaterial pro Oberflächeneinheit der im Wesentlichen kugelförmigen Teilchen, das ein Beschichtungslevel einer Zusammensetzung für die im Wesentlichen kugelförmigen Teilchen von Schritt (a) ergibt, das ein gewünschtes Freisetzungsprofil in vivo bereitstellt;

(c) Bestimmen der Oberfläche einer neuen Charge von im Wesentlichen kugelförmigen Teilchen, die den gleichen Wirkstoff enthalten, der in den im Wesentlichen kugelförmigen Teilchen enthalten war, die in Schritt (b) beschichtet wurden;

(d) Beschichten der neuen Charge von im Wesentlichen kugelförmigen Teilchen mit der in Schritt (b) aufgebrachten Beschichtung, um eine Beschichtung auf der neuen Charge von im Wesentlichen kugelförmigen Teilchen bereitzustellen, wobei die Beschichtung auf der neuen Charge von kugelförmigen Teilchen im Wesentlichen das gleiche Gewicht an Beschichtungsmaterial pro Oberflächeneinheit der im Wesentlichen kugelförmigen Teilchen hat, wie in Schritt (b) bestimmt.

2. Verfahren nach Anspruch 1, wobei das Gesamtgewicht der aufgebrachten Beschichtung zwischen 0,05 und 3 mg / $cm^2$ liegt.

3. Verfahren nach Anspruch 2, wobei das Gesamtgewicht der aufgebrachten Beschichtung zwischen 0,1 und 1 mg / $cm^2$ liegt.

4. Verfahren nach einem der vorhergehenden Ansprüche, wobei die Teilchengrößen zwischen 100 und 1400 Mikrometer als Durchmesser liegen.

5. Verfahren nach Anspruch 4, wobei die Teilchengrößen zwischen 600 und 1200 Mikrometer als Durchmesser liegen.

6. Verfahren nach Anspruch 5, wobei die Teilchengrößen zwischen 700 und 1100 Mikrometer als Durchmesser liegen.

7. Verfahren nach einem der vorhergehenden Ansprüche, wobei der Beta-blocker aus der Gruppe ausgewählt ist, die aus Propranolol, Metoprolol, Timolol, Oxpremolol, Alpremolol, Pindolol, Sotalol, Alebutolol und Atenolol besteht.

8. Verfahren nach Anspruch 7, wobei der Betablocker Propranololhydrochlorid ist.

**Revendications**

1. Procédé de revêtement de particules essentiellement sphériques comprenant un composé bêta-bloquant dans lequel les pellets sont pratiquement exempts de défauts de surface, avec un revêtement formant une membrane pour fournir une membrane sur lesdites particules essentiellement sphériques qui contrôle la libération du composé actif de ladite particule sensiblement sphérique, ledit procédé comprenant les étapes suivantes:

(a) déterminer la surface d'un échantillon pesé desdites particules essentiellement sphériques comprenant un composé bêta-bloquant dans un premier lot de particules sensiblement sphériques sur la base de la distribution granulométrique et de la densité;

(b) déterminer le poids de matériau de revêtement par unité de surface desdites particules essentiellement sphériques qui fournira un niveau de revêtement d'une composition pour lesdites particules sensiblement sphériques de l'étape (a) qui fournira un profil de libération souhaité in vivo;

(c) déterminer la surface d'un nouveau lot de particules essentiellement sphériques contenant le même composé actif que celui contenu dans les particules essentiellement sphériques qui ont été revêtues à l'étape (b);

(d) enrober ledit nouveau lot de particules essentiellement sphériques avec le revêtement appliqué à l'étape (b) pour former un revêtement sur ledit nouveau lot de particules essentiellement sphériques, ledit revêtement sur ledit nouveau lot de particules sphériques ayant essentiellement le même poids de matériau de revêtement par unité de surface des particules essentiellement sphériques, comme déterminé à l'étape (b).

2. Procédé selon la revendication 1, dans lequel le poids total de revêtement appliqué est compris entre 0,05 et 3 mg / $cm^2$.

3. Procédé selon la revendication 2, dans lequel le poids total de revêtement appliqué est compris entre 0,1 et 1 mg / $cm^2$.

**4.** Procédé selon l'une quelconque des revendications précédentes, dans lequel les tailles de particules sont comprises entre 100 et 1400 microns de diamètre.

**5.** Procédé selon la revendication 4, dans lequel les tailles de particules sont comprises entre 600 et 1200 microns de diamètre.

**6.** Procédé selon la revendication 5, dans lequel les tailles de particules sont comprises entre 700 et 1100 microns de diamètre.

**7.** Procédé selon l'une quelconque des revendications précédentes, dans lequel le bêta-bloquant est choisi dans le groupe comprenant le propranolol, le métoprolol, le timolol, l'oxprémolol, l'alprémolol, le pindolol, le sotalol, l'alébutolol et l'aténolol.

**8.** Procédé selon la revendication 7, dans lequel le bêta-bloquant est l'hydrochlorure de propranolol.

## REFERENCES CITED IN THE DESCRIPTION

**Patent documents cited in the description**

- US 5527545 A
- US 4138475 A
- US 6449869 B
- US 6354728 B
- US 20040185111 A

**Non-patent literature cited in the description**

- Aqueous Polymeric Coatings for Pharmaceutical Dosage Forms. Marcel Dekker. Inc, 1989, 350-355
- Pharmaceutical Pelletization Technology. Marcel Dekker, Inc, 1989, 246-252